# EUROPEAN PATENT SPECIFICATION

(11) **EP 0 391 909 B1**
(45) Date of publication and mention of the grant of the patent: **17.08.1994**
(21) Application number: 88908033.9
(22) Date of filing: 31.08.1988
(51) Int. Cl.: A61K 9/00, A61K 9/08, A61K 37/02

(54) **OCULAR CYCLOSPORIN COMPOSITION**
CYCLOSPORIN-AUGENMITTEL
COMPOSITION DE CYCLOSPORINE OCULAIRE

(30) Priority: 03.09.1987 US 92466; 04.11.1987 US 117218; 29.04.1988 US 187823
(43) Date of publication of application: 17.10.1990
(73) Proprietor: THE UNIVERSITY OF GEORGIA RESEARCH FOUNDATION, INC., Athens, Georgia 30602 (US)
(72) Inventor: KASWAN, Renee, Athens, GA 30605 (US)
(74) Representative: Bassett, Richard Simon
(86) International application number: PCT/US88/03039
(87) International publication number: WO 89/01772

(56) References cited:
- EP-A- 0 224 352
- WO-A-85/03640
- DE-A- 3 526 545
- US-A- 3 608 073
- US-A- 4 115 544
- US-A- 4 388 307
- US-A- 4 649 047
- Dialog Information Services, File 351: WPI 81-88, 51 accession no. 4331684 (WPI accession no. 86-335072), H. Mizushima: "Eyedrops composed of lipid microspheres contg. remedies for eye troubles", & JP-A-61249918, published 7 November 1986
- KLIN MBL Augenheilk., Vol. 187, 1985, F. Hoffman et al.: "Lokale Behandlung des Hornhauttransplantates beim Menschen mit Cyclosporin A", pp. 92-96

## Description

The present invention relates to a method and composition for increasing tear production by the topical administration of cyclosporin to the patient's eyes.

### BACKGROUND OF THE INVENTION

The exposed part of a normal eye is covered by a continuous thin tear film which is important for the well-being of the corneal and conjunctival epithelium and provides the cornea with an optically high quality surface. In addition, the aqueous part of the tear film acts as a lubricant to the eyelids during blinking of the lids. Certain enzymes contained in the tear fluid, for example immunoglobulin A, lysozyme and beta lysin, also have bacteriostatic properties.

The lacrimal apparatus consists of the secretory system (the source), the distribution system and the excretory system (the sink). In the secretory system, the bulk of the tear film, the aqueous tears, are supplied by the main and accessory lacrimal glands. The continuous production and drainage of aqueous tears is important in maintaining the corneal and conjunctival epithelium in a moist state, in providing nutrients for epithelial respiration, in supplying bacteriostatic agents and in cleaning the ocular surface by the flushing action of tear movement.

Abnormalities of the tear film include an absolute or partial deficiency in aqueous tear production, called keratoconjunctivitis sicca or KCS. In relatively mild cases, the main symptom of KCS is a foreign body sensation or a mild "scratchiness". This can develop into a constant, intense burning or irritative sensation which can be debilitating to the patient. More severe forms lead to the development of filamentary keratitis, a painful condition characterized by the appearance of numerous strands or filaments attached to the corneal surface. Recent evidence suggests that these filaments represent breaks in the continuity of the normal corneal epithelial cells. The shear created by lid motion pulls these filaments, causing pain. Management of this stage of KCS is very difficult.

A frequent complication of KCS is secondary infection. Several breakdowns in the eye's normal defense mechanism seem to occur, presumably attributable to a decrease in the concentration of antibacterial lysozyme in the aqueous tears of a patient suffering from KCS.

Although KCS can develop in the absence of any other overt systemic abnormality, there is a frequent association of KCS with systemic disease. KCS can occur as part of a larger systemic involvement known as Sjogren's syndrome which is characterized by dry eyes, dry mouth, and arthritis.

Histologically in KCS (as part of Sjogren's syndrome or in isolation), the initial changes seen in the lacrimal gland are those of focal lymphocytic and plasma cell infiltrates associated with degeneration of glandular tissue. These changes resemble those seen in autoimmune disease in other tissue, giving rise to the speculation that KCS has an autoimmune basis.

Sjogren's syndrome is recognized as an exocrine glad dysfunction. Characteristically, the lacrimal glands show a mononuclear-cell infiltration that ultimately leads to destruction of the glandular structure.

Conventional treatment of KCS is symptomatic. Normally, aqueous-deficient dry eye states are treated by supplementation of the tears with artificial tear substitutes. However, relief is limited by the retention time of the administered artificial tear solution in the eye. Typically, the effect of an artificial tear solution administered to the eye dissipates rapidly, within about thirty to forty-five minutes. The effect of such products, while soothing initially, does not last long enough. The patient is inconvenienced by the necessity of repeated administration of the artificial tear solution in the eye as needed to supplement the normal tears. Moreover, such treatment merely acts to alleviate the symptoms of the dry eye state and does not cure any underlying disorders or causes of the dry eye state.

The systemic use of corticosteroids has been advocated to treat these conditions. However, the merit of systemic corticosteroids in dry eye states has not been established. In most dry eye cases the hazards of long-term use of antiinflammatory agents would seem to outweigh their potential merit. It has also been suggested to administer orally a dilute solution of pilocarpine to stimulate the autonomic nervous system to effect increased aqueous tear production. This method of treatment has not met with universal favor because of the unpleasant side effects of ingested pilocarpine.

Surgical procedures have also been suggested in the management of dry eye states. Where there has been significant conjunctival destruction, mucous membrane transplants have been advocated. It has also been suggested that parotid (saliva) duct transplantation can be useful in the management of dry eyes. However, since surgical alterations to combat dry eye conditions constitute such a drastic remedy and the benefit resulting from these alterations is questionable, these methods are usually used only as a last resort.

Cyclosporin is a metabolite isolated from the culture broths of the fungal species Tolypocladium inflatum Gams. A neutral, hydrophobic cyclic peptide composed of eleven amino acid resides, cyclosporin includes a previously unknown N-methylated amino acid composed of nine carbon atoms. Wenger, Synthesis of Cyclosporin and Analogues, pp. 14-25 in Cyclosporin A 1, Grune & Stratton, Inc. (New York 1983). A number of additional cyclosporins (B, C, D, E, and G) have been reported since the first cyclosporin was isolated (CsA). As described in U.S. Patent No. 4,117,118 issued September 26, 1978 to Harri et al., cyclosporin is readily soluble in most of the usual organic solvents and practically insoluble in petroleum ether and water. As distributed by Sandoz Ltd., Basel, Switzerland, under the tradename Sandimmune, cyclosporin for oral administration is dissolved in olive oil for further dilution with food and in polyoxyethylated castor oil and ethanol for intravenous injection.

Cyclosporin A was first proposed for use as an antifungal agent, but its immunosuppressive effects were found to be more marked than its antifungal potential. A potent immunosuppressive agent, cyclosporin is used to prolong survival of allogeneic transplants involving skin, heart, kidney, pancreas, bone marrow, small intestine and lung. The exact mechanism of action is not known but experimental evidence suggests that the effectiveness of cyclosporin is due to specific and reversible inhibition of immunocompetent cells, primarily T-helper cells. Lymphokine production, gamma interferon production and release of interleukin-2 or T-cell growth factor are also inhibited by cyclosporin.

Cyclosporin's immunosuppressive properties have led to its use in immune system related diseases. For example, U.S. Patent No. 4,649,047 describes a method for the treatment of phacoanaphylactic endophthalmitis and uveitis in the anterior or posterior segment of an eye wherein cyclosporin is topically administered to the eye. In other ophthalmic applications, cyclosporin has been used topically only for the treatment of external (e.g., corneal) eye diseases.

BenEzra et al., Amer. J. Ophthalmol. 101: 278-282 (1986), describe the effect of 2% cyclosporin eyedrops on severe vernal keratoconjunctivitis. Severe vernal keratoconjunctivitis is a seasonal allergic disorder unrelated to tear deficiency.

Hunter et al., Clin. Exp. Immunol. 45: 173-177 (1981) describe the topical administration of cyclosporin in a rabbit model of corneal graft rejection with positive results.

Boisjoly et al., Arch. Ophthalmol. 102: 1804-1807 (1984), have reported that topical application of cyclosporin had a beneficial prophylactic effect towards the treatment of severe herpetic stromal keratitis.

Mosteller et al., Investigative Ophthalmol. Supp. 25, 3: 38 (1984), disclose treating corneal allograft rejection in rabbits by applying a single dose of a 10% Cyclosporin A ointment in the lower cul-de-sac of the eyelids.

Cyclosporin has also been used systemically in other ophthalmic applications, where the disease being treated is not limited to the eye surface. For example, Nussenblatt et al., Amer. J. Ophthalmol. 96: 275-282 (1983), have reported clinical improvement in some patients with noninfectious posterior uveitis following systemic treatment with cyclosporin.

Cyclosporin is primarily administered orally or by injection. Unfortunately, cyclosporin used systemically has been associated with a high incidence of renal toxicity (kidney failure), some cases of hepatotoxicity, increased incidence of lymphoid tumors and increased incidence of opportunistic infections. Cyclosporin is only slightly less toxic than other immunosuppressive agents such as cytoxan or aziothioprine. The systemic side effects of cyclosporin are so severe and so common that they limit its use to life-threatening or in some cases severe sight-threatening disease. Finally, systemic application of cyclosporin is limited by its prohibitive cost.

As described in U.S. Patent No. 4,649,047 issued March 10, 1987 to Kaswan, topical administration of cyclosporin is useful in the treatment of a variety of immune mediated disorders of the eye, including uveitis and phacoanaphylactic endophthalmitis. This is also the preferred mode of administration to avoid the undesirable side effects and cost of systemic administration.

To date, there has been no suggestion to treat a glandular dysfunction, a lacrimal gland dysfunction or an aqueous-deficient dry eye state with a cyclosporin, either topically or systemically.

Although cyclosporin has been topically administered in a variety of vehicles including arachis oil, a commercially available ointment base, and castor oil, the conventional carrier is olive oil. Unfortunately, topical administration of cyclosporin in olive oil to the eye of either humans or dogs is frequently accompanied by a burning sensation, pain, and redness. In some cases, other side effects have been observed including lid edema and periocular alopecia (hair loss around the eye). Similar problems have occurred with topical ophthalmic use of cyclosporin in the other vehicles. Studies have now demonstrated that these unpleasant side effects are due to the carrier, not to the cyclosporin. Unfortunately, cyclosporin is of very limited solubility and the number of acceptable carriers for ophthalmic use is limited.

It is therefore an object of this invention to provide a method of increasing tear production for a normal or tear-deficient eye, regardless of cause.

It is another object of this invention to provide a cyclosporin-based treatment of lacrimal gland dysfunction without the accompanying adverse physiological responses and economic difficulties associated with systemic cyclosporin treatments.

It is another object of the present invention to provide a composition containing an effective concentration of cyclosporin for topical ophthalmic use which does not cause burning, redness or irritation.

It is a still further object of the present invention to provide a composition for topical ophthalmic use which is stable upon storage.

It is still another object of the present invention to provide a composition for topical ophthalmic use which promotes normal healing of the epithelial surface of the eye.

### SUMMARY OF THE INVENTION

The present invention is directed to a method of treating a dry eye state in a patient by administering a cyclosporin topically to the patient's eye. The treatment is useful regardless of the cause of the dry eye, and includes treatment of autoimmune dysfunction of the lacrimal glands. The treatment is also useful in the enhancement or restoration of normal tear production, and normal healing of the surface of the eye.

The preferred composition for topical administration to the eye consists of cyclosporin dissolved in corn oil. The composition may further include antioxidants, lubricants, antibiotics, antifungals, antivirals, pilocarpine, vasoconstrictors, surfactants, wetting agents, anti-inflammatory agents (i.e. corticosteroids), preservatives, mucolytic agents (i.e. bromhexine, acetylcysteine), as well as other compounds.

The most preferred composition is 2% cyclosporin, 1 mole % alpha tocopherol and 0.005% methyl paraben in corn oil.

### Brief Description of the Drawings

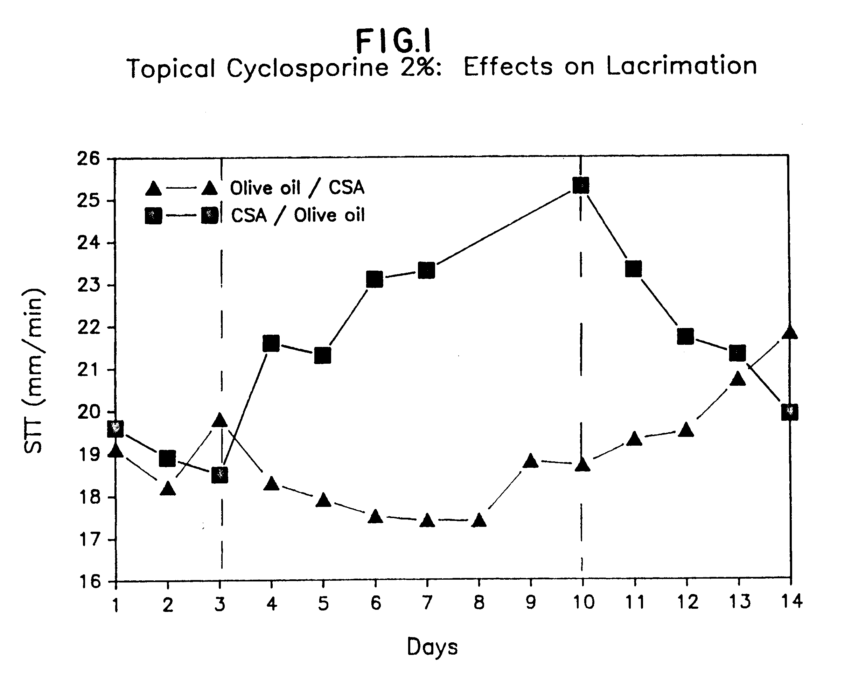

Figure 1 is a graph demonstrating the effect of topical cyclosporin on lacrimination (STT mm/min) over time (days) in twelve normal male beagle dogs; following three days of baseline measurement with no treatment, six dogs were treated with 2% cyclosporin in olive oil applied topically two times daily, and six dogs were treated with placebo (olive oil) applied topically two times daily. The STT were determined twice daily in the cyclosporin treated dogs ( ■----■) and in the olive oil treated dogs ( ▲ --- ▲ ). Following 7 days all dogs were crossed over into the opposite treatment groups for an additional three days.

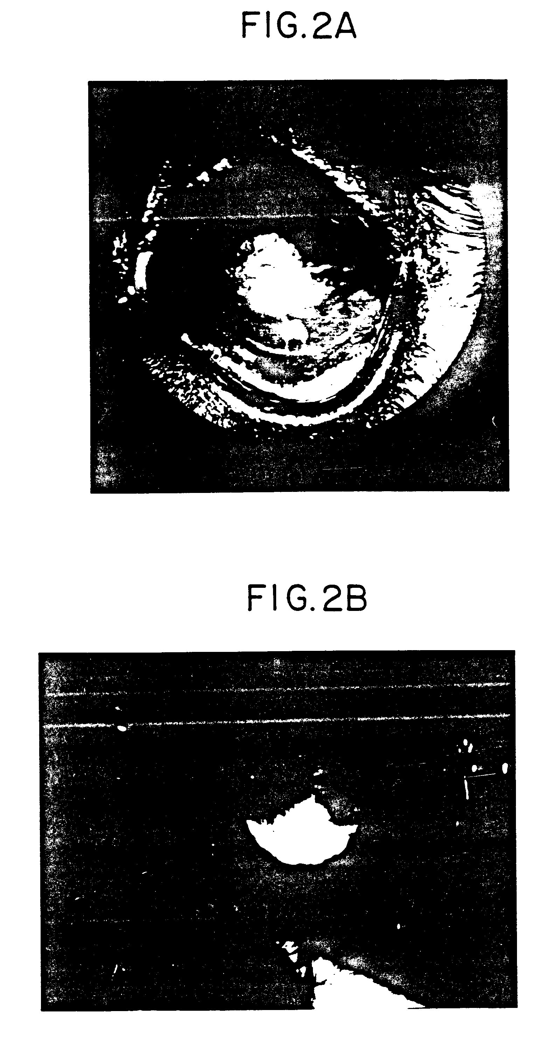

Figure 2 is a comparison of the appearance of the eye of a dog suffering from keratoconjunctivitis sicca before (Figure 2A) and after (Figure 2B) treatment for four weeks with 2% cyclosporin.

### DETAILED DESCRIPTION OF THE INVENTION

The present invention provides a method of treating tear-deficient dry eyes due to autoimmune disease or of unknown etiology which includes the step of administering a cyclosporin topically to the patient's eye. The invention includes a corn oil based cyclosporin composition which provides greatly enhanced benefits when applied topically to the eye over previous cyclosporin compositions.

Despite the apparent similarities in chemical structure, studies demonstrate the significant differences in comfort and incidence of side effects between cyclosporin in previously described carriers such as olive oil and cyclosporin in corn oil, both with and without preservative and antioxidant. These studies also establish that topically applied cyclosporin can be used to promote or effect normal healing and prevent or reverse scar formation on the ocular surface.

In accordance with the present invention, the cyclosporin may be used in any efficacious concentration, e.g., 0.01 to saturation (e.g., up to 50 weight percent of a cyclosporin) in a pharmaceutically acceptable excipient. Concentrations of 1.1 to 20 weight percent of a cyclosporin are preferred. Although the preferred vehicle is corn oil, as described below, other pharmaceutically acceptable excipients are, for example, animal oil, vegetable oil, appropriate organic or aqueous solvents, artificial tear solutions in which the cyclosporin is soluble, and natural or synthetic polymers or appropriate membranes.

Examples of these pharmaceutically acceptable excipients are olive oil, arachis oil, castor oil, mineral oil, petroleum jelly, dimethyl sulfoxide, chremophor, Miglyol 182 (commercially available from Dynamit Nobel Kay-Fries Chemical Company, Mont Vale, New Jersey), alcohol (e.g., ethanol, n-propyl alcohol or iso-propyl alcohol), liposomes or liposome-like products, silicone fluids and mixtures thereof.

Examples of artificial tear excipients which can be advantageously used in the practice of this invention are isotonic sodium chloride, cellulose ethers such as hydroxypropylmethylcellulose and hydroxyethylcellulose, polyvinyl alcohol and other commercially available artificial tear solutions. An example of a useful polymeric excipient is polyoxyethylated castor oil. Examples of pharmaceutically acceptable membranes which can advantageously be used in the practice of this invention are: microdone, an artificial lipid membrane, polyvinylalcohol, or methylcellulose.

Cyclosporins which are useful in the practice of the present invention include both natural or synthetic cyclosporins. Cyclosporin A is preferred in the practice of the present invention. Other forms of cyclosporins (e.g., analogs and isomers such as Cyclosporins B, C, D, E, and H) may also be used. Mixtures of different cyclosporins may be used.

In the preferred method of treating a specific antigen mediated immune response in a patient having more than one involved site of the immune mediated response, the cyclosporin is applied locally to each involved site. For example, where only one eye appears to suffer from immune mediated KCS, both eyes should be treated. Surprisingly, unless each involved site is treated with cyclosporin, no appreciable benefit is obtained from cyclosporin treatment at any one of the sites, i.e., if only the affected eye is treated, little benefit of the cyclosporin is obtained. This suggests that locally administered cyclosporin interferes with and blocks the afferent immune recognition of the specific antigen which triggers the immune mediated response. Unless each of the sites wherein the specific antigen occurs is treated with the cyclosporin, T-cell antigen signaling occurs continuously in the untreated site causing local lymphokine production that triggers a cascading systemic immune response that adversely affects both the treated and untreated sites.

In other words, local administration of cyclosporin acts to inhibit the continuous afferent immune response, for example within an eye, when antigens placed in the eye are associated with intraocular MHC antigen bearing accessory cells and are presented to T-cells. However, if an untreated eye or a distal skin graft is used to initiate antigen recognition, an afferent immune response begins at the site distal to the treatment site, T-cells are activated, lymphokines produced, and the systemic immune response proceeds to an efferent immune response affecting both eyes.

Thus, in any experiment in which an intrasubject control eye is used, local therapy in one eye will have diminished effect unless it is given in such quantity as to produce systemic immuno-suppression. Moreover, when treating an immune mediated response which exists to a much greater degree at one site of a patient, such as in or near one eye of the patient, than at another site of the patient, such as in the other eye of the patient, so as to be apparent at only the one site, the cyclosporin is advantageously administered locally in a therapeutically effective amount to each of the sites. An immune mediated response which appears to exist in only one eye of a patient is advantageously treated by administering a therapeutically effective amount of a cyclosporin locally to both eyes of the patient to achieve maximal benefit.

If administered locally to each of the sites of the patient affected by the immune mediated response, cyclosporin can be used advantageously to treat a variety of immune mediated disorders. Ocular diseases which are successfully treated in animal models by local administration of cyclosporin to each site of the patient effected by the immune mediated response include immune-mediated melting ulcers in cats and dogs, chronic neovascularization and proliferative keratitis in cats and dogs, stromal keratitis subsequent to ulcerative Herpes keratitis in cats, pigmentary keratitis in dogs and KCS in dogs.

Numerous advantages accrue with the practice of the present invention. The method of the present invention is useful in that it can locally prevent activation of a pre-systemic response. Topical administration of a cyclosporin into a patient's tear deficient eye increases tear production in the eye. Thus, such treatment further serves to correct corneal and conjunctival disorders exacerbated by tear deficiency and KCS, such as corneal scarring, corneal ulceration, inflammation of the cornea or conjunctiva, filamentary keratitis, mucopurulent discharge and vascularization of the cornea. Furthermore, cyclosporin directly decreases the immune response of granulation and neovascularization in the cornea.

Further objects of this invention, together with additional features contributing thereto and advantages accruing therefrom, will be apparent from the following examples of the invention.

### Example 1: Effectiveness and distribution of topical cyclosporin.

Topical administration to a patient's eye has surprisingly been found to be an excellent method for providing a cyclosporin to the lacrimal glands of the patient to treat KCS. Additionally, since by its very nature topical administration does not require cyclosporin dispersion throughout the patient's system as is the case with systemic administration, the present invention provides a means for directing cyclosporin to the desired location without the accompanying high risk of adverse responses and high cost associated with systemic treatments.

Cyclosporin concentration has been determined for various eye compartments and tissues surrounding the eye after bilateral topical administration of cyclosporin to the eyes of three rabbits. The cyclosporin was administered in each of the rabbits' eyes in drops (approximately 17 microliters) of 2% radiolabelled cyclosporin in an olive oil solution applied every 15 minutes for 6 applications, followed by a period of two hours to allow for absorption. The rabbits were then euthanized and the eyes and surrounding tissue enucleated and frozen. The eyes and surrounding tissue were dissected into their component parts. These were then digested in collagenase and the resulting solutions analyzed by liquid scintillation counting for cyclosporin content. The following average cyclosporin concentrations were measured:
Accessory lacrimal gland: 2850 ng of cyclosporin/gram of tissue;
Periorbital fat: 800 ng/gram;
Cornea: 4700 ng/gram;
Iris: 1200 ng/gram;
Retina: 50 ng/gram;
Aqueous humor: 30 ng/gram;
Vitreous humor: 30 ng/gram;
Anterior sclera: 3150 ng/gram; and
Posterior sclera: 1550 ng/gram.

### Example 2: Comparison of treatment of dry eye with anti-inflammatory steroid and cyclosporin.

A one year old standard female Poodle with conjunctivitis exhibited mild aqueous tear deficiency in both eyes. The dog had a Schirmer tear test value of 15 mm/minute in the right eye and 10 mm/minute in the left eye.

The Schirmer tear test is a test of aqueous tear production. The test depends upon observing the extent of wetting of a strip of filter paper placed over the lower lid of an eye for a specified time. Standardized strips are commercially available. The strip is folded at a notched marking and is then placed over the edge of the lateral one-third of the eyelid. The strip is usually left in place for a period of time while the patient looks straight ahead in dim light.

The degree of wetting of the paper is measured in mm from the notch. For human patients, a normal end point is 5 mm of wetting at five minutes. For canine patients, the normal tear production is 14 to 20 mm of wetting at one minute.

The dog was originally treated with dexamethasone by topical administration in both eyes four times daily. Although initially somewhat effective, treatment was subsequently discontinued and the same dog at approximately six years old still exhibited conjunctivitis in both eyes and had a Schirmer tear test value of 3 mm/minute in both eyes. Topical dexamethasone was then applied to both eyes twice daily for nine weeks without benefit.

The dog was then treated by topical application of 2% cyclosporin in an olive oil solution in both eyes once daily without any other medications. After ten days, the dog showed markedly increased tear production and had a Schirmer tear test value of 22 mm/minute in the right eye and 8 mm/minute in the left eye.

The treatment by topical application of 2% cyclosporin in an olive oil solution in both eyes once daily was continued for an additional three weeks. At this time, the dog exhibited plentiful aqueous tear production and the treatment was stopped for one week. After the one week, the dog had a Schirmer tear test value of 10 mm/minute in the right eye and 9 mm/minute in the left eye.

At this time, the treatment by topical application of 2% cyclosporin in an olive oil solution in both eyes once daily was reinstituted and continued for six days. After the six days, the dog had a Schirmer tear test value of 22 mm/minute in the right eye and 16 mm/minute in the left eye.

In this case, a dog with chronic tear deficiency in which prior use of corticosteroids failed to improve tear secretion showed a surprising increase in tear production with cyclosporin treatment. The increased tear production continued only while cyclosporin therapy continued. When the treatment was stopped for a week, the eyes again became tear deficient. However, tear production increased to normal levels after the treatment was restarted.

### Example 3: Treatment of dry eye and enhancement of corneal healing with topical cyclosporin treatment.

An eight year old male Lhasa Apso had a four year old cat scratch in his left eye and an active 4 mm stromal ulcer in his right eye. An ocular examination of the dog showed conjunctivitis in both eyes with mucopurulent discharge, diffuse irregular corneal surfaces, pigment formation and neovascularization in the cornea of the left eye. The Schirmer tear test values were 12 mm/minute in the right eye and 3 mm/minute in the left eye.

The dog was treated with topical administration to both eyes of 2% cyclosporin in an olive oil solution once daily, neosporin twice daily and ophthalmic petrolatum. After five days, the Schirmer tear test values were 22 mm/minute in the right eye and 23 mm/minute in the left eye. In addition, the ulcer in the right eye was healed to 2 mm and the left eye was assessed to have decreased vascularization.

In this case, cyclosporin increased tear production significantly in a short period of time. Moreover, cyclosporin, unlike corticosteroids, did not retard corneal healing nor activate corneal collagenase. Accordingly, cyclosporin can be used in eyes having active corneal ulcers.

### Example 4: Comparison of treatment of dry eye with pilocarpine alone and in combination with cyclosporin.

A six year old male English Bulldog with a long history of KCS had Schirmer tear test values of 2 mm/minute in the right eye and 3 mm/minute in the left eye.

The right eye was neovascularized over the entire cornea. No intraocular detail could be visualized through the opaque cornea. The cornea was grossly thick and irregular in surface. The left eye had neovascularization over about half of the cornea, mostly axially.

The dog was treated with three drops of 2% pilocarpine by mouth. After two hours, the Schirmer tear test values were 0 mm/minute in the right eye and 10 mm/minute in the left eye.

The dog was then treated with 2% cyclosporin in an olive oil solution administered topically to both eyes once daily and three drops of 2% pilocarpine administered by mouth twice daily. After twelve days, the Schirmer tear test values were 10 mm/minute in the right eye and 15 mm/minute in the left eye.

In this case, while pilocarpine alone increased tear production in the left eye from a Schirmer tear test value of 3 mm/minute to 10 mm/minute, pilocarpine did not increase tear production in the right eye. Use of cyclosporin with pilocarpine increased tear production to a Schirmer tear test value of 15 mm/minute in the left eye and from 0 mm/minute to 10 mm/minute in the right eye. The use of cyclosporin markedly increased tear production over the use of pilocarpine alone.

### Example 5: Correction of dry eye and restoration of normal vision by topical treatment with cyclosporin.

A seven year old Miniature Poodle had a history of severe KCS of six to seven months duration. Treatment with artificial tears six times daily did not affect the apparent blindness.

The dog showed marked mucopurulent discharge in both eyes. The Schirmer tear test values were 0 mm/minute in both eyes. The dog's corneas were thickened and neovascularized with an irregular surface. No intraocular detail could be visualized through the opaque corneas.

The dog was treated with one drop of 2% pilocarpine by mouth two times daily and ophthalmic petrolatum four times daily. After two weeks, the Schirmer tear test values were still 0 mm/minute in both eyes. The corneal vascularity and scarring remained dense and the anterior chambers of the dog's eye were not visualizable.

The dog was then treated with 2% cyclosporin in an olive oil solution administered topically in both eyes once daily and two drops pilocarpine administered by mouth twice daily.

After two weeks, the Schirmer tear test values were 8 mm/minute in the right eye and 6 mm/minute in the left eye. Although corneal vascularization and scarring remained, the iris and lens could be evaluated, there was no mucoid discharge in either eye as previously and the KCS was assessed as medically improved.

After similar treatment for another two months, the Schirmer tear test values were 11 mm/minute in the right eye and 17 mm/minute in the left eye. The dog's eyes had minimal corneal vascularization and minimal scarring.

In this case, although the dog was treated initially with pilocarpine, pilocarpine alone is not known to cause such a drastic improvement in tear production. After treatment with cyclosporin, the dog improved from no tear flow in either eye to normal tear production in both eyes. The dog improved from blinding corneal inflammation to very mild corneal pigmentation in both eyes. Treatment with cyclosporin markedly increased tear production and allowed the dog to return to normal vision.

### Example 6: Stimulation of tearing in normal dogs.

Studies were conducted on the effect of applying topical 2% cyclosporin in olive oil to the eyes of normal dogs. The results are shown in Figure 1 comparing the effect of topical cyclosporin on lacrimination in six normal male beagle dogs, before and after several days of olive oil therapy alone. In both studies, no treatment was given on days 1 to 3 to establish a baseline. On days 4-10, as graphed by the triangles, one drop of olive oil was administered twice daily (BID) to each eye. On days 11-13, one drop of 2% cyclosporin in olive oil was administered twice daily. A significant increase in tearing was observed. On days 4-10, as graphed by the squares, one drop of 2% cyclosporin in olive oil was administered twice daily. On days 11-13, one drop of olive oil was applied to each eye twice daily. The significant increase in tearing observed over days 4-10 persisted through days 11-13 in the absence of cyclosporin treatment.

The data conclusively demonstrate that topically applied cyclosporin increases glandular function, i.e., lacrimination, in normal eyes.

Since cyclosporin has very low solubility in most solutions which can be administered to the eye, the cyclosporin in the majority of studies on the efficacy of topical administration of cyclosporin has been suspended in olive oil. Unfortunately, controlled studies comparing olive oil alone and in combination with cyclosporin demonstrate that the vehicle, the olive oil, produces redness and burning. In animals, pain is evidenced by the animal holding its eyes shut. In approximately 5 to 10% of approximately 1000 months of treatment (based on number of bottles of 2% cyclosporin dispensed for veterinary use where one bottle is sufficient for treatment of an animal twice daily for about one month), other side effects were observed, including lid edema, corneal surface irregularities, and periocular alopecia.

The present invention includes the surprising discovery that corn oil can be substituted for olive oil as the vehicle for topical administration of cyclosporin to the eye to avoid the undesirable side effects due to the use of the olive oil. Over 3000 bottles of 2% cyclosporin have now been dispensed for treatment of animals twice daily without any apparent side effects for periods of time up to four months.

Additives to the corn oil which enhance stability of the cyclosporin solution include antioxidants such as alpha tocopherol and preservatives such as methyl paraben. Other antioxidants are known to those skilled in the art. There are some indications that alpha tocopherol (Vitamin E) may also have beneficial effects on the eye since oxidative radicals increase inflammatory damage. Preliminary clinical observations on the protective action of oral administration of vitamins A and E on the corneal epithelium were recently published by Gerhardinger, et al., in Acta Vitaminol. Enzymol. 7 (Supp),71-74 (1985). Other compounds which may be added to the cyclosporin solution include emollients, viscosity modifying agents, antioxidants, preservatives, antibiotics, antifungals, antivirals, lubricants, surfactants, vasoconstrictors, DMSO, parasympathomimetics, cholinergics, neurotransmitters, lacrimogenic agents, substance P agonists, substance P antagonists, mucolytics, prostaglandin antagonists, lipogenase inhibitors, cyclooxygenase inhibitors, antiinflammatories, oxygen scavengers, hydrating agents, and epitheliotropic agents. Specific examples, in addition to alpha tocopherol and methyl paraben, include vitamin A, retinoic acid, pilocarpine, hyaluronic acid, polyvinyl alcohol, methylcellulose, eledoisin, physalaemin, bromhexine, mucosolvan, acetylcysteine, indomethacin, and corticosteroids.

The most preferred formulation at this time for topical ophthalmic use consists of 2% cyclosporin, 1 mole % alpha tocopherol and 0.005% methyl paraben. However, cyclosporin solutions can be prepared of between approximately 0.01% by weight and saturation, approximately 20% by weight. Unless otherwise specified, all percentages of compounds herein are by weight.

Although the usual means of administration of the compound is by administration of cyclosporin drops to the surface of the eye, delayed or prolonged release of the cyclosporin at a selected site can also be achieved by encapsulating the cyclosporin-oil mixture within a polymeric implant, liposomes, or microcapsules. Methods for making polymeric implants for ocular use are taught by U.S. Patent No. 3,960,150 to Hussain et al. Both non-degradable and biodegradable polymers can be used, including polyethylene, polystyrene, polypropylene, polyanhydrides, polyorthoester, polylactic acid, and polyglycolic acid. Methods for encapsulating materials within liposomes are taught by PCT/US85/00220 publication WO 85/03640 29 August 1985 by the Liposome Company. Methods for encapsulation of biological material within microcapsules for-implantation are taught by U.S. Patent No. 4,352,883 to Lim. Other suitable methods and materials are known to those skilled in the art.

The following non-limiting examples demonstrate the efficacy and advantages of topical cyclosporin in corn oil for treatment of immune disorders, enhancement or restoration of tear production, and enhancement or effecting of normal healing of the surface of the eye.

### Example 6: Stimulation of tearing in normal dogs.

Studies were conducted on the effect of applying topical 2% cyclosporin in olive oil to the eyes of normal dogs. The results are shown in Figure 1 comparing the effect of topical cyclosporin on lacrimination in six normal male beagle dogs, before and after several days of olive oil therapy alone. In both studies, no treatment was given on days 1 to 3 to establish a baseline. On days 4-10, as graphed by the triangles, one drop of olive oil was administered twice daily (BID) to each eye. On days 11-13, one drop of 2% cyclosporin in olive oil was administered twice daily. A significant increase in tearing was observed. On days 4-10, as graphed by the squares, one drop of 2% cyclosporin in olive oil was administered twice daily. On days 11-13, one drop of olive oil was applied to each eye twice daily. The significant increase in tearing observed over days 4-10 persisted through days 11-13 in the absence of cyclosporin treatment.

The data conclusively demonstrate that topically applied cyclosporin increases glandular function, i.e., lacrimination, in normal eyes.

### Example 7: Topically applied cyclosporin: Lacrimomimetic effects and reduction of corneal scars in dogs with KCS.

Twenty five cases (22 bilateral, 2 unilateral cases) of spontaneous KCS were treated with a solution of 2% cyclosporin (CsA) in olive oil, 1 gtt QD - BID, OU, and evaluated for changes in tear production as determined by Schirmer tear test (STT) and for changes in the surface of the globe.

The effects of cyclosporin were twofold: cyclosporin increased tear production in 84% of idiopathic cases of canine KCS and cyclosporin caused marked regression of corneal pathology including superficial granulation tissue, neovascularization and pigmentation, without retarding healing of corneal ulcers. Case histories are summarized in Table 1.

The diagnosis of KCS preceded CsA use by 0-60 months, with an average of 1.1 yr. Prior treatment included artificial tears in 16/25 dogs, oral or topical pilocarpine in 11/25 dogs, oral or topical corticosteroids in 11/25 dogs, topical antibiotics in 9/25 dogs, or no prior treatment in 5/25 dogs.

Contrary to expectation, the longevity of KCS did not correlate inversely with response to therapy. The average STT before administration of cyclosporin was 2.54 mm/min right eye and 2.46 mm/min left eye. During the period in which cyclosporin eyedrops were administered, the mean STT value was 11.38 mm/min right eye and 11.50 mm/min left eye. The average increase in STT was 8.84 mm/min right eye (t = 7.5 Student's T -test for related measures, p<0.0005), and 9.04 mm/min left eye (t = 6.7, p<0.0005). 38 eyes were initially diagnosed as having severe KCS (STT 0-4 mm/min). Following treatment, STT values increased by greater than 5 mm/min in 84% of severely affected eyes. Dogs were noted to have increased STT beginning 3 to 56 days after onset of cyclosporin therapy. Of the six eyes (6/38, 16%) determined to be nonresponsive, five were evaluated for only a short period (7 to 35 days). Because STT value in responsive eyes increased with increased frequency and duration of treatment (see Table I, cases 21 and 22), the 84% success rate may be an underestimate.

In six dogs whose STT values increased in response to cyclosporin, treatment was discontinued and the STT values regressed. When cyclosporin was reinstituted, the STT increased back to maximal levels in six hours in one case, and in 1-7 days in the other four cases. In two dogs receiving cyclosporin on alternate days, the STT values decreased on nontreatment days. Even with sporadic interruptions in administration of cyclosporin treatment, no dog has lost responsiveness to cyclosporin. Many of the cyclosporin responsive dogs previously had been unresponsive to corticosteroids administered topically, subconjunctivally, and parenterally.

In dogs with superficial corneal granulation tissue, continuous use of cyclosporin resulted in a progressive decrease in the abnormal thickness and opacity of the cornea. Even in dogs that did not have an increase in tear secretion, alleviation of the corneal disease was generally marked. Most dogs with dense blinding pigmentation and superficial granulation had marked clearing of the corneas after several months of treatment. Three dogs had corneal ulcers at the onset of treatment with cyclosporin; each healed within 48 hours of onset of treatment. Dogs maintained for prolonged periods (8-12 months) relapsed into KCS within 2-3 days of withdrawal of cyclosporin.

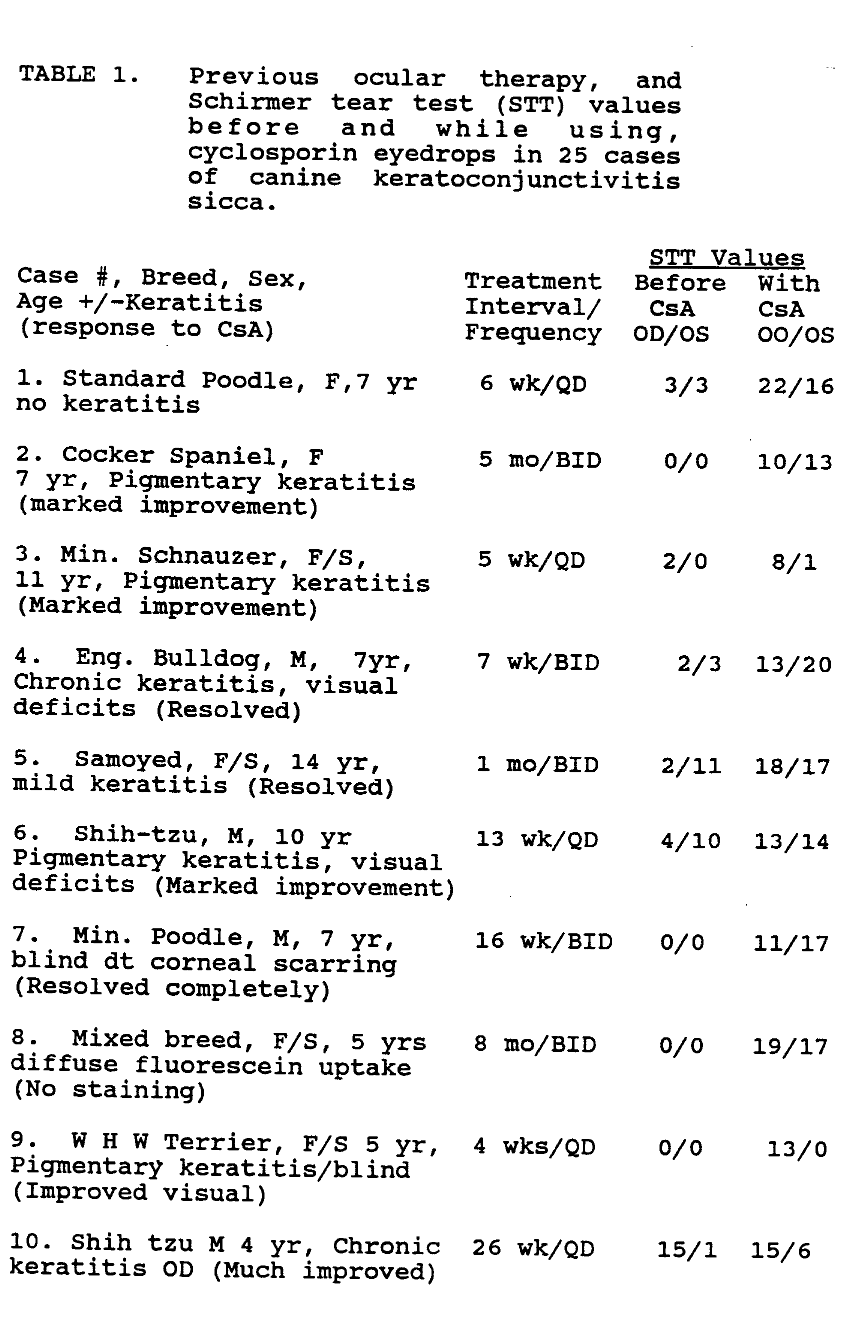

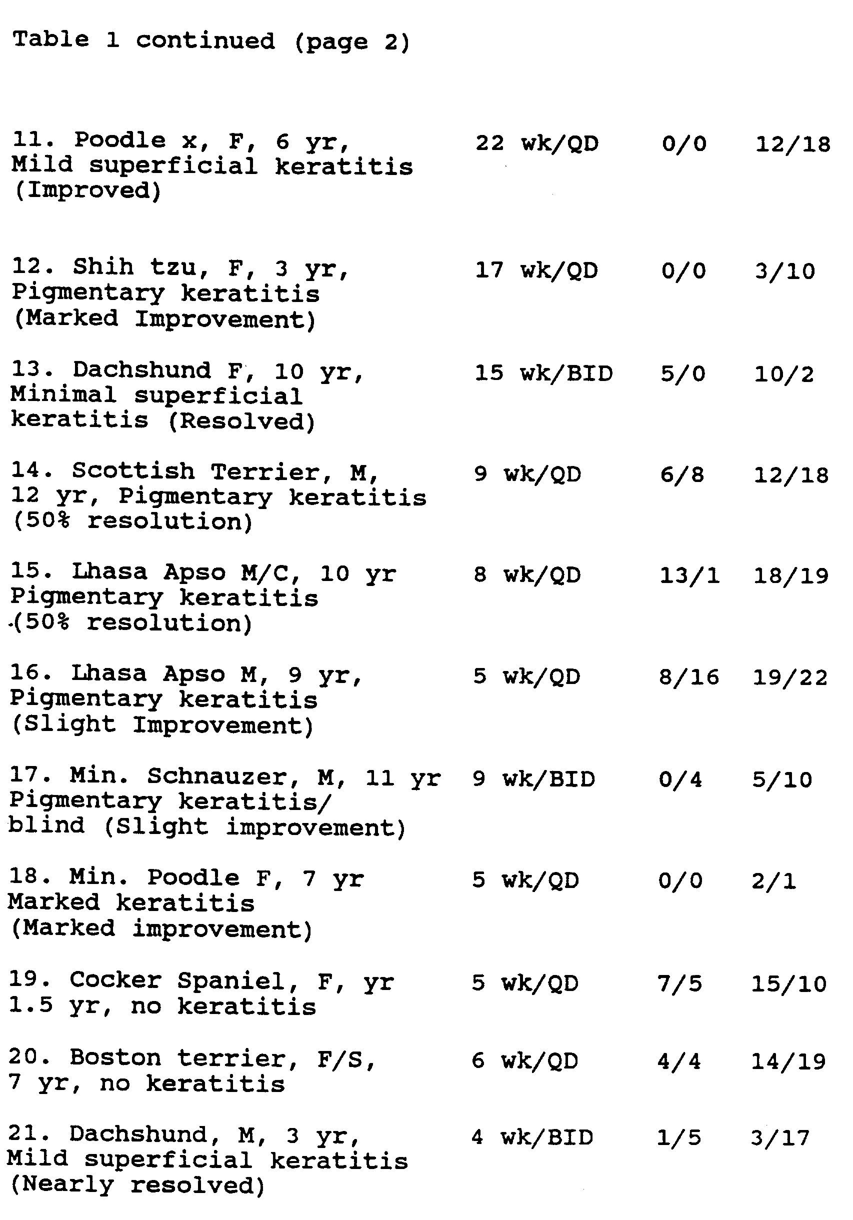

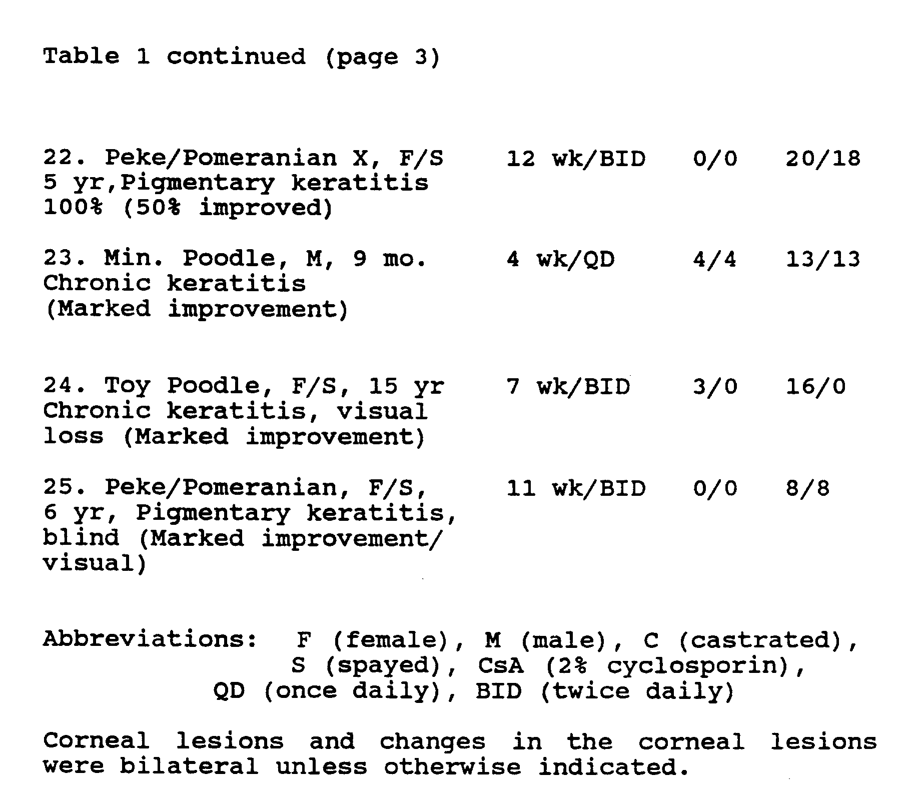

### Example 8: Stimulation of tearing in humans suffering from Sjogren's syndrome.

Sjogren's syndrome is characterized by chronic infiltration of the exocrine glands, principally the lacrimal and salivary glands, by mononuclear leukocytes. The process causes the progressive destruction of the glandular tissue and is characterized by the development of keratoconjunctivitis sicca (KCS), or "dry eye". Neither topical nor parenteral treatment using steroids has been completely effective in decreasing irritation of the corneal surface nor in preventing corneal ulcer formation. In fact, topical or parenteral corticosteroids do not enhance lacrimation and can retard healing of corneal ulcers and are therefore considered to be contraindicated by many ophthalmologists.

A human patient with primary Sjogren's syndrome (dry eye with dry mouth) was treated with topical 2% cyclosporin in corn oil. The patient had been treated for years with conventional therapy, artificial tears Q 15 mins. For the past several months his STT were 2-3 mm/5min/eye. (In humans the STT is measured for 5 minutes, unlike the dog where it is measured for only 1 min. However, the expected normal values are the same, i.e., normal is 14 mm, values under 5 mm are indicative of a severe case of dry eye).

Following 9 days of twice daily therapy of both eyes, his STT was 20 and 23 mm/5 min/eye, a significant increase over the pretreatment values. Prior to treatment, the corneas had stained diffusely in both eyes with fluorescein dye, an indication of corneal ulcers. After 9 days of therapy, one eye had no staining and one eye only stained over 1/3 of the surface.

Three women with severe chronic secondary Sjogren's syndrome were treated for 1 week with BID 2% cyclosporin in corn oil containing 1 mole% alpha tocopherol and 0.005%methyl paraben. All three had abnormal corneas. The first had a nonhealing corneal ulcer which penetrated the full thickness of the surface epithelium covering the cornea. This ulcer healed within two days of onset of therapy. The second had a "contact lens cornea", an indentation at the circumference of the cornea which gives it the appearance of an eye wearing a contact lens, when no lens is present, which is analogous to a scar. The indentation showed evidence of filling in within 7 days of therapy. The third had corneal lesions which also showed improvement within one week. All had increases in the STT.

The results conclusively demonstrate the effectiveness of topically administered cyclosporin in alleviating the symptoms of KCS, promoting normal healing and actually reducing scar tissue on the surface of the eye.

### Example 9: Promotion of normal healing of the eye surface without restoration of normal tearing in a dog.

An 11-year old spayed Miniature Schnauzer had been determined to have KCS 8 months before admission. Analysis of a specimen obtained by conjunctival scraping at that time revealed distemper virus. The dog had been treated with 2% pilocarpine, (1 gtt PO q 12 h) which initially caused an increase in the STT to 8 mm/min bilaterally but later lost efficacy, as the STT decreased to 0 mm/min bilaterally. Treatment had been dexamethasone ointment Q 12 h bilaterally, artificial tears approximately 6 times daily, and petrolatum ointment at bedtime. On admission, ophthalmic observation showed the STT to be 2 mm/min right eye, 0 mm/min left eye, with mucoid conjunctivitis bilaterally, dorsal corneal pigmentation of approximately 40-50% of the corneal surface, and superficial neovascularization extending approximately 6 mm into the dorsal half of the cornea bilaterally (Fig. 2B). The corneal surfaces were modeled irregularly but translucent, and there were no apparent visual deficits. Complete blood count and serum thyroxin were normal and the only abnormality detected on serum profile was an elevated serum alkaline phosphatase (667 mg/dl).

Cyclosporin (2% 1 gtt QD, bilaterally) was prescribed, with artificial tears to be administered as needed. In 4 weeks, the STT had increased to 8 mm/min in the right eye but was still 0-1 mm/min in the left eye. The conjunctivitis had improved, but was still evident in the left eye. However, there was marked improvement of the corneal surface bilaterally (Fig. 2B).

A parotid duct transposition was performed at this time and the lacrimal gland of each third eyelid biopsied. Microscopically both glands were similar, with diffuse often intense periductal and interstitial infiltration of plasma cells and lymphocytes, and fibrosis of the acini and tubules. Focal areas of normal acinar tissue were seen in each gland, and some areas contained dilated tubules lined with flattened epithelium. The results in the preceding examples establish that topically applied cyclosporin can be used to resolve corneal ulcers even in the absence of restoration of tearing. As dramatically shown by Figure 2A and 2B, the eye surface becomes clearer, smoother, and vision is improved.

### Example 10: Comparison of olive oil and corn oil vehicles for cyclosporin for topical ophthalmic use.

Among the animals treated with cyclosporin in olive oil, within four days of beginning treatment, four dogs and one cat had ocular irritation reactions including: hyperemia of the bulbar conjunctiva, corneal surface irregularities with apparent corneal edema, and blepharospasms indicative of ocular pain.

In each case therapy was withdrawn and these symptoms resolved. Therapy with cyclosporin in corn oil was begun in three of the dogs following resolution of the ocular irritation reactions. All three dogs tolerated the corn oil/cyclosporin mixture well.

In the fourth dog, cyclosporin in olive oil was used less frequently than the BID prescription because the owner thought the drug irritated the eyes, but kept using it on an infrequent basis. Following two to three weeks of use, bilateral periocular alopecia occurred and the lids were intensely hyperemic. The cyclosporin in olive oil was discontinued for several weeks. Cyclosporin in corn oil was begun BID bilaterally. The lesions of chronic corneal vascularization and superficial keratitis resolved markedly, the STT increased, and there was no recurrence or irritation or alopecia.

Olive oil and corn oil were also compared in normal, human eyes. The olive oil produced a burning sensation lasting 15 to 60 minutes. The corn oil produced a milder sensation lasting only 1 to 2 minutes.

No side effects have been noted in the any of the 3000 bottles of 2% cyclosporin in corn oil dispensed for animal use, in comparison with the 5 to 10% incidence of side effects in 1000 bottles of 2% cyclosporin in olive oil dispensed for animal use. The substantial difference in tolerance of the two oils is surprising since the chemical nature of olive oil and corn oil is very similar. Tests of the levels of free fatty acids and pH do not indicate any significant differences which could account for the decreased tolerance for olive oil. Substitution of purified olive oil, Sigma Chemical Co., St. Louis, MO, or first press olive oil, for the Berio brand olive oil, obtained from the grocery store, which was used initially, does not eliminate the irritation.

## Claims

1. A topical ophthalmic composition comprising a corn oil base containing between 0.01% and saturation cyclosporin.

2. A composition characterised in that the composition is adapted for only topical ophthalmic, to the exclusion of oral and injectable administration and comprising a corn oil base containing between 0.01% and saturation cyclosporin.

3. The composition of Claim 1 or 2 further comprising a compound selected from the group consisting of emollients, viscosity modifying agents, antioxidants, preservatives, antibiotics, antifungals, antivirals, lubricants, surfactants, vasoconstrictors, DMSO, parasympathomimetics, cholinergics, neurotransmitters, lacrimogenic agents, substance P agonists, substance P antagonists, mucolytics, prostaglandin antagonists, lipogenase inhibitors, cyclooxygenase inhibitors, anti-inflammatories, oxygen scavengers, hydrating agents, and epitheliotropic agents.

4. The composition of Claim 3 wherein the compound is selected from the group consisting of vitamin A, vitamin E, retinoic acid, pilocarpine, hyaluronic acid, polyvinyl alcohol, methylcellulose, methyl paraben, eledoisin, physalaemin, bromhexine, mucosolvan, acetylcysteine, indomethacin, and corticosteroids.

5. The composition of Claim 1 or 2 comprising 2% cyclosporin in corn oil.

6. The composition of Claim 5 further comprising a compound selected from the group consisting of alpha tocopherol and methyl paraben.

7. The composition of Claim 1 or 2 wherein said composition is encapsulated.

8. The composition of Claim 7 wherein said composition is encapsulated within a polymeric matrix.

9. The composition of Claim 8 wherein said composition is encapsulated within a polymeric matrix formed of a polymer selected from the group consisting of polyethylene, polystyrene, polypropylene, polyanhydrides, polyorthoester, polylactic acid, and polyglycolic acid.

10. The composition of Claim 7 wherein said composition is encapsulated within liposomes.

11. The composition of Claim 7 wherein said composition is microencapsulated.

12. The composition of Claim 1 or 2 wherein said cyclosporin is in a dosage which promotes normal wound healing.

13. The composition of Claim 1 or 2 wherein said cyclosporin is in a dosage which stimulates or restores lacrimal gland activity.

14. The composition of Claim 1 or 2 wherein said cyclosporin is in a dosage which suppresses an immune disorder.

15. Use of cyclosporin in the manufacture of a topical ophthalmic composition for restoring or enhancing lacrimal gland function.

16. Use of cyclosporin of claim 15, said composition comprising a solution of cyclosporin in a corn oil base.

## Patentansprüche

1. Topisches Augenmittel, umfassend eine Maisölgrundlage mit zwischen 0,01% und dem Sättigungswert an Cyclosporin.

2. Mittel, das lediglich für eine topische Anwendung im Auge vorgesehen ist und dessen orale Verabreichung und Verabreichung durch Injektion ausgeschlossen sind, umfassend eine Maisölgrundlage mit zwischen 0,01% und dem Sättungswert an Cyclosporin.

3. Mittel nach Anspruch 1 oder 2, zusätzlich enthaltend eine Verbindung, ausgewählt aus der Gruppe weichmachende Mittel, viskositätsmodifizierende Mittel, Antioxidanzien, Konservierungsmittel, Antibiotika, Mittel gegen Pilze, antivirale Mittel, Gleitmittel, oberflächenaktive Mittel, gefäßverengende Mittel, DMSO, Parasympathomimetika, Cholinergika, Neurotransmitter, tränenerzeugende Mittel, Substanz P-Agonisten, Substanz P-Antagonisten, schleimlösende Mittel, Prostaglandin-Antagonisten, Lipogenaseinhibitoren, Cyclooxygenaseinhibitoren, entzündungshemmende oder -widrige Mittel, Sauerstoffänger, Hydratisierungsmittel und epitheliotrope Mittel.

4. Mittel nach Anspruch 3, wobei die Verbindung aus der Gruppe Vitamin A, Vitamin E, Retinoesäure, Pilocarpin, Hyaluronsäure, Polyvinylalkohol, Methylcellulose, Methylparaben, Eledoisin, Physalaemin, Bromhexin, Mucosolvan, Acetylcystein, Indomethacin und Corticosteroide ausgewählt ist.

5. Mittel nach Anspruch 1 oder 2, enthaltend 2% Cyclosporin in Maisöl.

6. Mittel nach Anspruch 5, zusätzlich enthaltend eine Verbindung, ausgewählt aus der Gruppe α-Tocopherol und Methylparaben.

7. Mittel nach Anspruch 1 oder 2 in eingekapselter Form.

8. Mittel nach Anspruch 7, eingekapselt in einer polymeren Matrix.

9. Mittel nach Anspruch 8, welches in einer aus einem Polymeren, ausgewählt aus der Gruppe Polyethylen, Polystyrol, Polypropylen, Polyanhydride, Polyorthoester, Polymilchsäure und Polyglycolsäure, gebildeten polymeren Matrix eingekapselt ist.

10. Mittel nach Anspruch 7, eingekapselt in Liposomen.

11. Mittel nach Anspruch 7 in mikroeingekapselter Form.

12. Mittel nach Anspruch 1 oder 2, wobei das Cyclosporin in einer eine normale Wundheilung fördernden Dosis vorhanden ist.

13. Mittel nach Anspruch 1 oder 2, wobei das Cyclosporin in einer die Tränendrüsenaktivität stimulierenden oder wiederherstellenden Dosis vorhanden ist.

14. Mittel nach Anspruch 1 oder 2, wobei das Cyclosporin in einer eine Immunstörung unterdrückenden Dosis vorhanden ist.

15. Verwendung von Cyclosporin bei der Herstellung eines topischen Augenmittels zur Wiederherstellung oder Verstärkung der Tränendrüsenfunktion.

16. Verwendung von Cyclosporin nach Anspruch 15, wobei das Mittel eine Lösung von Cyclosporin in einer Maisölgrundlage umfaßt.

## Revendications

1. Composition ophtalmique topique comprenant une base d'huile de maïs contenant de la cyclosporine entre 0,01 % et la saturation.

2. Composition caractérisée en ce que la composition est adaptée seulement pour l'administration ophtalmique topique, à l'exclusion de l'administration orale et injectable, et qu'elle comprend une base d'huile de maïs contenant de la cyclosporine entre 0,01 % et la saturation.

3. Composition selon la revendication 1 ou 2, comprenant en outre un composé choisi dans le groupe constitué des émollients, des agents modifiant la viscosité, des antioxydants, des conservateurs, des antibiotiques, des antifongiques, des antiviraux, des lubrifiants, des agents tensioactifs, des vasoconstricteurs, du DMSO, des parasympathomimétiques, des cholinergiques, des neurotransmetteurs, des agents lacrymogènes, des agonistes de la substance P, des antagonistes de la substance P, des mucolytiques, des antagonistes des prostaglandines, des inhibiteurs de la lipogénase, des inhibiteurs de la cyclooxygénase, des anti-inflammatoires, des désoxygénants, des agents hydratants, et des agents épithéliotropes.

4. Composition selon la revendication 3, dans laquelle le composé est choisi dans le groupe constitué de la vitamine A, de la vitamine E, de l'acide rétinoïque, de la pilocarpine, de l'acide hyaluronique, de l'alcool polyvinylique, de la méthylcellulose, du méthylparabène, de l'élédoïsine, de la physalaémine, de la bromhexine, du mucosolvane, de l'acétylcystéine, de l'indométhacine, et des corticostéroïdes.

5. Composition selon la revendication 1 ou 2, comprenant 2 % de cyclosporine dans de l'huile de maïs.

6. Composition selon la revendication 5, comprenant en outre un composé choisi dans le groupe constitué de l'alpha tocophérol et du méthylparabène.

7. Composition selon la revendication 1 ou 2, dans laquelle ladite composition est encapsulée.

8. Composition selon la revendication 7, dans laquelle ladite composition est encapsulée dans une matrice polymère.

9. Composition selon la revendication 8, dans laquelle ladite composition est encapsulée dans une matrice polymère formée d'un polymère choisi dans le groupe constitué du polyéthylène, du polystyrène, du polypropylène, des polyanhydrides, du polyorthoester, de l'acide polylactique, et de l'acide polyglycolique.

10. Composition selon la revendication 7, dans laquelle ladite composition est encapsulée dans des liposomes.

11. Composition selon la revendication 7, dans laquelle ladite composition est microencapsulée.

12. Composition selon la revendication 1 ou 2, dans laquelle ladite cyclosporine est à une posologie qui favorise une cicatrisation normale des blessures.

13. Composition selon la revendication 1 ou 2, dans laquelle ladite cyclosporine est à une posologie qui stimule ou restaure l'activité des glandes lacrymales.

14. Composition selon la revendication 1 ou 2, dans laquelle ladite cyclosporine est à une posologie qui supprime un trouble immunitaire.

15. Utilisation de la cyclosporine dans la fabrication d'une composition ophtalmique topique pour restaurer ou stimuler la fonction des glandes lacrymales.

16. Utilisation de la cyclosporine selon la revendication 15, ladite composition comprenant une solution de cyclosporine dans une base d'huile de maïs.
